## Europäisches Patentamt

## European Patent Office

## Office européen des brevets

(11) Veröffentlichungsnummer: **0 134 291**
**B1**

(12)

# EUROPÄISCHE PATENTSCHRIFT

(45) Veröffentlichungstag der Patentschrift:
13.08.86

(21) Anmeldenummer: 83108838.0

(22) Anmeldetag: 07.09.83

(51) Int. Cl.⁴: **C 12 Q 1/40, C 12 Q 1/44**

(54) Rekonstituierbares Trockenreagenz für diagnostische Zwecke und Verfahren zu seiner Herstellung.

(43) Veröffentlichungstag der Anmeldung:
20.03.85 Patentblatt 85/12

(45) Bekanntmachung des Hinweises auf die Patenterteilung:
13.08.86 Patentblatt 86/33

(84) Benannte Vertragsstaaten:
AT BE CH DE FR GB IT LI LU NL SE

(56) Entgegenhaltungen:
EP-A-0 014 252
EP-A-0 021 572
EP-A-0 065 348
WO-A-83/00676
US-A-4 036 697

(73) Patentinhaber: **AMERICAN HOSPITAL SUPPLY CORPORATION, One American Plaza, Evanston, IL 60201 (US)**

(72) Erfinder: **Danninger, Josef, Finkenstrasse 27a, D-8032 Gräfelfing (DE)**
Erfinder: **Spaethe, Reiner, Dr., Prinz- Karl- Strasse 41, D-8130 Starnberg- Söcking (DE)**
Erfinder: **Lampart, Alfred, Dr., Obere Egg 2, CH-4312 Magden (CH)**
Erfinder: **Tenger, Franziska, Oberrothen, CH- 3154 Rüschegg (CH)**

(74) Vertreter: **Brose, D. Karl, Dipl.- Ing., Patentanwälte Brose & Partner Wiener Strasse 2, D-8023 München- Pullach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

## Beschreibung

Die Erfindung bezieht sich auf ein rekonstituierbares Trockenreagenz für diagnostische Zwecke mit wenigstens einem proteinhaltigen Bestandteil sowie weiteren Bestandteilen. Sie hat auch ein Verfahren zur Herstellung dieses Trockenreagenzes zum Gegenstand.

Bei der Lipasebestimmung wird bekanntlich primär die α-Esterbindung des Triglycerids unter Bildung des Diglycerids und einer freien Fettsäure hydrolisiert. Diese Hydrolyse kann titimetrisch oder turbidimetrisch verfolgt werden.

Für die Diagnostik der Pankreaserkrankung besitzt die Bestimmung der Enzyme Lipase und Amylase eine hohe Aussagekraft. Bei akuter Pankreatitis kommt es nämlich zu einem starken Anstieg beider Enzyme im Serum. Die Amylase wird jedoch wegen ihres geringen Molekulargewichts durch die Niere relativ schnell wieder ausgeschieden, während die Lipaseaktivität im Serum längere Zeit erhöht bleibt. Eine sichere Diagnose der Pankreatitis ist durch die Bestimmung beider Enzyme zu erhalten.

Damit die Lipase ihre maximale Aktivität entfalten kann, benötigt sie Co-Lipase als Co-Faktor. Da die Co-Lipase jedoch ca. zweimal schneller als Lipase von der Niere aus dem Serum entfernt wird, muß dem lipasehaltigen Serum Co-Lipase bei der Lipasebestimmung zugesetzt werden.

Aus der DE—As 29 04 305 ist ein Trockenreagenz zur Lipasebestimmung bekannt, das 0,2 bis 10 Gewichtsprozent Triglycerid, 20 bis 90 Gewichtsprozent Schutzkolloid, 5 bis 60 Gewichtsprozent Gallensäurealkalisalz, 0,001 bis 0,1 Gewichtsprozent Co-Lipase, 0,1 bis 2,0 Gewichtsprozent Konservierungsmittel, 5 bis 20 Gewichtsprozent Harnstoff, 3 bis 50 Gewichtsprozent Puffersubstanz für pH 6,0 bis 10,5 und 0,5 bis 5 Gewichtsprozent Aktivator enthält. Zur Herstellung dieses Reagenzes wird eine wässrige Emulsion durch Lösen des Gallensäuresalzes, der Co-Lipase sowie wenigstens eines Teils des Schutzkolloids und des Konservierungsmittels und anschließendes Einspritzen einer Triglyceridlösung in diese Lösung gebildet. Anschließend wird die Emulsion lyophilisiert, worauf die restlichen Bestandteile, nämlich die Puffersubstanz, Harnstoff sowie das restliche Schutzkolloid und das restliche Konservierungsmittel sowie der Aktivator zugegeben werden.

Nach dem bekannten Verfahren läßt sich also ein zufriedenstellendes Trockenreagenz nur durch Lyophilisation der meisten Bestandteile sowie durch Zugabe von Fremdstoffen, wie Harnstoff, erhalten.

Auch ist es bekannt, zur Amylasebestimmung ein rekonstituierbares Reagenz zu verwenden, das aus einem Lyophilisat besteht, das α-Glucosidase und Oligosaccharid enthält. Dieses Reagenz ist mit einem relativ großen Leerwert behaftet.

Aufgabe der Erfindung ist es, ein einwandfreies rekonstituierbares Trockenreagenz für diagnostische Zwecke, das wenigstens einen proteinhaltigen Bestandteil sowie weitere, nichtproteinhaltige Bestandteile enthält, anzugeben, dessen Herstellung mit einem relativ geringen Aufwand verbunden ist.

Diese Aufgabe wird durch das im Anspruch 1 gekennzeichnet Reagenz bzw. das im Anspruch 13 gekennzeichnete Verfahren gelöst.

Bei dem erfindungsgemäßen Reagenz wird also ein Lyophilisat, das den proteinhaltigen Bestandteil enthält, mit einem Granulat, das die weiteren Bestandteile enthält, zu einem Monoreagenz, d.h. zu einem durch Wasserzugabe gebrauchsfertigen Reagenz kombiniert. Bei der Bestimmung von Enzymen liegt dabei das Substrat in aller Regel in dem Granulat vor, während in dem Lyophilisat als Proteine insbesondere Indikatorenzyme oder C-Enzyme enthalten sind.

Das Granulat, das die nichtproteinhaltigen Bestandteile enthält, kann nach dem erfindungsgemäßen Verfahren unter schärferen Bestimmungen hergestellt werden. So wird nicht nur die Lyophilisation als relativ aufwendige Trocknungsmethode umgangen, vielmehr können die nichtproteinhaltigen Bestandteile inniger miteinander vermischt und sehr fein zerkleinert werden. Aufgrund der unterschiedlichen Stabilität von Lyophilisat und Granulat ergeben sich auch insofern Produktvorteile, derart, daß das Granulat als der wesentlich stabilere Teil auf Vorrat produziert werden kann und bei der Herstellung einer Reagenziencharge nur das proteinhaltige Lyophilisat frisch zubereitet werden muß.

Weiterhin vereinfacht ein solches Vorgehen die Herstellung verschiedenster Abpackungsgrößen der Teste, da hierzu nur die Einwaage an Granulat geändert werden braucht. Beispielsweise ist Co-Lipase wenig thermostabil und durch Scherkräfte leicht zerstörbar. Durch die getrennte Zubereitung der weiteren Bestandteile, als des Triglycerids, der Emulgatoren usw. zu einem Granulat, kann also bei der Granulatherstellung eine erheblich höhere Temperatur angewendet werden, als dies bei der Lyophilisation der Fall ist. Ferner können die weiteren Bestandteile innig bei Raumtemperatur miteinander vermischt werden und anschließend scharf getrocknet werden, was eine sehr feine Zerkleinerung des Granulats möglich macht. Auf diese Weise wird die Bildung einer sehr stabilen Triglycerid-Emulsion guter Reproduzierbarkeit gewährleistet.

Wie vorstehend erwähnt, weist ein α-Glucosidase sowie Oligomeres enthaltendes Lyophilisat, das als Trockenreagenz zur Amylasebestimmung verwendet wird, einen relativ hohen Leerwert auf.

Es hat sich nun gezeigt, daß sich dieser Leerwert drastisch reduzieren läßt, wenn die Glucosidase einer separaten Lyophilisation bei einer maximalen Temperatur von 30 bis 50°C unterworfen wird.

Der hohe Leerwert des bekannten Trockenreagenzes ist wahrscheinlich darauf zurückzuführen, daß die Glucosidase enzymatische Verunreinigungen aufweist, die auch langkettigere Oligosaccharide spalten. Diese Spaltung vollzieht sich auch, wenn die Glucosidase und die Oligosaccharide vor der gemeinsamen Lyophilisation gelöst werden.

Wird hingegen nach einer bevorzugten erfindungsgemäßen Ausführungsform eine separate Lyophilisation der Glucosidase bei einer maximalen Temperatur von 30 bis 50°C durchgeführt, so gelingt es, diese enzymatischen Verunreinigungen zu inaktivieren, d.h. eine Glucosidase hoher Spezifität bereitzustellen.

Weiterhin weist das Reagenz nach der DE—AS 29 04 305 eine relativ lange Lag-Phase von vier Minuten und mehr auf. Von Bedeutung ist nun, daß sich nach einer bevorzugten Ausführungsform der Erfindung die Lag-Phase bei der Lipasebestimmung erheblich dadurch herabsetzten läßt, wenn statt einer Gallensäure ein Gemisch aus 1 bis 40 Gewichtsprozent Taurodesoxycholsäure, 2 bis 20 Gewichtsprozent Cholsäure und 5 bis 50 Gewichtsprozent Desoxycholsäure, jeweils in Form ihrer Alkalisalze, eingesetzt wird. Dadurch kann eine Herabsetzung der Lag-Phase auf zwei Minuten oder weniger erreicht werden.

Weiterhin wird durch dieses Gallensäuregemisch eine sichere Hemmwirkung gegenüber Postheparinlipase erzielt. Die Postheparinlipase wird aus dem Gewebe freigesetzt, wenn z.B. bei einer Operation Heparin verabreicht wird. Die Postheparinlipase stammt also nicht vom Pankreas und führt daher zu einer Verfälschung der Werte der Pankreaslipasebestimmung.

Als Triglycerid eignen sich für die Lipasebestimmung natürliche wie synthetische Triglyceride mit Fettsäureresten zwischen 4 und 22 C-Atomen. Beispielsweise erweist sich Tributyrin als geeignet. Bevorzugt werden jedoch Triglyceride mit längerkettigen ungesättigten Fettsäureresten, insbesondere Triglyceride, deren Fettsäurereste 8 bis 20 C-Atome und 1 bis 4, vorzugsweise 1 bis 2 Kohlenstoff-Kohlenstoff-Doppelbindungen aufweisen, verwendet. Aufgrund seiner leichten Zugänglichkeit wird Triolein besonders bevorzugt, auch Olivenöl ist sehr gut geeignet. Als Konservierungsmittel werden bei dem Reagenz zur Lipasebestimmung beispielsweise Alkaliazide, insbesondere Natriumazid, verwendet.

Als Puffersubstanz eignet sich für das Reagenz für die Lipasebestimmung beispielsweise Diäthanolaminpuffer, Triäthanolaminpuffer, Trispuffer usw. Ein besonders guter Aktivator ist Calciumchlorid.

Bei der Amylasebestimmung liegt in dem Testreagenz beispielsweise α-Glucosidase, Hexokinase (HK), Glucose - 6 - Phosphat - Dehydrogenase (G-6-PDH) sowie ein Oligomeres mit 4 bis 10 Glucoseeinheiten und Adenosintriphosphat (ATP) vor. Die Amylase im Serum hydrolisiert dabei das Oligosaccharid zu Maltose, die durch die α-Glucosidase in zwei Glucosemoleküle gespalten wird. Die freigesetzte Glucose wird beim U/V-Test mit HK und G-6-PDH bestimmt. Die HK bewirkt die Umsetzung der Glucose mit ATP unter Bildung von Adenosindiphosphat (ADP) und die G-6-PDH die Umsetzung von Glucose-6-Phosphat und NAD zu 6-Phosphogluconat und NADH, dessen Bildung verfolgt wird.

Als Puffer kann bei der Amylasebestimmung z.B. Phosphatpuffer eingesetzt werden. Als Aktivator sind Natriumchlorid und Magnesiumacetat geeignet.

Statt durch den UV-Test kann das erfindungsgemäße Reagenz auch zur Bestimmung der Amylase mittels Farbtest ausgebildet sein. Dazu wird als Oligosaccharid eine chromophore Gruppe aufweisendes Saccharid entsprechend der DE—OS 28 22 364 verwendet.

Die nachstehenden Beispiele dienen der weiteren Erläuterung der Erfindung.

Beispiel 1

In ein Reagenzgefäß (50 ml) werden 0,1 ml einer 0,4 gewichtsprozentigen Co-Lipaselösung lyophilisiert.

In 150 ml Wasser werden 34 mg Triolein, 480 mg Tris, 7,5 mg Calciumchlorid und 517 mg eines Gemischs aus 31 Gewichtsprozent Natrium-Taurodesoxicholat, 14 Gewichtsprozent Natrium-Cholat und 55 Gewichtsprozent Natrium-Desoxycholat gemischt. Das Gemisch wird im Rotationsverdampfer bei 70°C zur Trockene eingeengt, zerkleinert und mit einem Sieb mit einer Maschenweite von 0,25 mm gesiebt.

Von diesem Granulat werden 53 mg in das Reaktionsgefäß mit dem Lyophilisat eingewogen.

Zur Rekonstitution bzw. Herstellung der Testlösung werden dem Reaktionsgefäß 50 ml Aqua dest. gegeben.

Zu 2 ml dieser Testlösung werden 0,1 ml unverdünnte Serumprobe gegeben. Die Kalkulation erfolgt über einen Standard mit definierter Lipaseaktivität nach Rick (Z. klin. Chem. u. klin. Biochem., 1969, Heft 5, Seiten 530 bis 539).

Beispiel 2

In einem Reaktionsgefäß (50 ml) werden 0,05 ml einer α-Glucosidase-Lösung (280 KU/l), 0,15 ml Hexokinase-Lösung (830 KU/l) und 0,15 ml Glucose-6-Phosphat-Dehydrogenase (830 KU/l) lyophilisiert.

In 60 ml Wasser werden 3.500 mg Oligosaccharid (4 bis 10 Glucoseeinheiten), 8.520 mg Phosphatpuffer, 2.900 mg Natriumchlorid, 535 mg Magnesiumacetat, 862 mg Adenosin-Triphosphat (ATP) und 1.989 mg Nicotinamid-Adenin-Dinucleotid (NAD) gemischt. Das Gemisch wird in einem Rotationsverdampfer bei 70°C unter Vakuum eingedampft. Der trockene Rückstand wird zerkleinert und mit einem Sieb mit einer Maschenweite von 0,25 mm gesiebt. Von diesem Granulat werden 915 mg in das Reagenzgefäß mit dem Lyophilisat eingewogen. Zur Herstellung der Testlösung werden 50 ml Aqua dest. dem Reaktionsgefäß zugegeben. Zu 1 ml dieser Testlösung wird 0,05 ml unverdünnte Serumprobe gegeben. Die Kalkulation erfolgt über den Extinktionskoeffizienten des durch die Nachweisreaktion gebildeten NADH.

**Patentansprüche**

1. Rekonstituierbares Trockenreagenz für diagnostische Zwecke mit wenigstens einem proteinhaltigen Bestandteil sowie weiteren Bestandteilen, dadurch gekennzeichnet, daß der proteinhaltige Bestandteil als Lyophilisat vorliegt und die weiteren Bestandteile als Granulat.

2. Reagenz nach Anspruch 1 zur Lipasebestimmung unter Verwendung von 0,001 bis 0,2 Gewichtsprozent Co-Lipase und 0,2 bis 10 Gewichtsprozent eines emulgierbaren Triglycerids, dadurch gekennzeichnet, daß die Co-Lipase im Lyophilisat vorliegt und das Triglycerid im Granulat.

3. Reagenz nach Anspruch 2, dadurch gekennzeichnet, daß das Granulat außerdem 10 bis 60 Gewichtsprozent Puffersubstanz für pH 6,0 bis 10,5, 5 bis 60 Gewichtsprozent Gallensäuren-Alkalisalz, 0,1 bis 2,0 Gewichtsprozent Konservierungsmittel und 0,5 bis 5 Gewichtsprozent Aktivator enthält.

4. Reagenz nach Anspruch 3, dadurch gekennzeichnet, daß im Granulat 10 bis 60 Gewichtsprozent Gallensäuren-Alkalisalz vorliegt und das Gallensäuren-Alkalisalz im Granulat durch ein Gemisch aus 1 bis 40 Gewichtsprozent Taurodesoxycholsäure, 2 bis 20 Gewichtsprozent Cholsäure und 10 bis 70% Desoxycholsäure in Form ihrer Alkalisalze gebildet ist.

5. Reagenz nach einem der Ansprüche 2 bis 4, dadurch gekennzeichnet, daß der Aktivator durch ein Chlorid, vorzugsweise Calciumchlorid gebildet wird.

6. Reagenz nach Anspruch 1 zur Amylasebestimmung unter Verwendung von α-Glucosidase oder α- und β-Glucosidase und Oligosacchariden, dadurch gekennzeichnet, daß die α-und/oder β-Glucosidase im Lyophilisat vorliegt und die Oligosaccharide im Granulat.

7. Reagenz nach Anspruch 6, dadurch gekennzeichnet, daß es 50 bis 500 U/l α- und/oder β-Glucosidase sowie 0,03 bis 7 mmol/l Oligosaccharide, jeweils bezogen auf das rekonstituierte Reagenz enthält.

8. Reagenz nach Anspruch 6 oder 7, dadurch gekennzeichnet, daß das Granulat zusätzlich 3 bis 50 Gewichtsprozent Puffersubstanz für pH 6 bis 10 und 0,5 bis 5 Gewichtsprozent Aktivator enthält.

9. Reagenz nach einem der Ansprüche 6 bis 8 zur Bestimmung der Amylase mittels UV-Test, dadurch gekennzeichnet, daß im Lyophilisat außerdem Hexokinase und Glucose-6-Phosphat-Dehydrogenase und im Granulat zusätzlich Adenosin-5'-Triphosphat vorliegt.

10. Reagenz nach einem der Ansprüche 6 bis 8 zur Bestimmung der Amylase mittels Farbtest, dadurch gekennzeichnet, daß das Oligosaccharid eine chromophore Gruppe besitzt.

11. Reagenz nach Anspruch 10, dadurch gekennzeichnet, daß das Oligosaccharid aus 4 bis 10 Glucoseeinheiten besteht und als chromophore Gruppe p-Nitrophenol aufweist.

12. Verfahren zur Herstellung eines rekonstituierbaren Trockenreagenzes nach einem der vorstehenden Ansprüche, dadurch gekennzeichnet, daß in ein Reaktionsgefäß der proteinhaltige Bestandteil bzw. die Co-Lipase bzw. die α- und/oder β-Glucosidase als Lyophilisat und die weiteren, nicht-proteinhaltigen Bestandteile als Granulat eingebracht werden, wobei das Granulat durch Lösen, Suspendieren oder Emulgieren der weiteren Bestandteile und anschließendes Trocknen nach einem anderen Trocknungsverfahren als der Lyophilisation erhalten wird.

13. Verfahren nach Anspruch 12, dadurch gekennzeichnet, daß das Lyophilisat durch Lyophilisieren einer Lösung, Suspension oder Emulsion des proteinhaltigen Bestandteils bzw. der Co-Lipase bzw. der α- und/oder β-Glucosidase in dem Reaktionsgefäß hergestellt und das Granulat zugewogen wird.

14. Verfahren nach Anspruch 12 oder 13, dadurch gekennzeichnet, daß das Trocknen der weiteren Bestandteile im Vakuum bei Raumtemperatur oder erhöhter Temperatur erfolgt.

15. Verfahren nach Anspruch 12 zur Herstellung eines Reagenz zur Amylasebestimmung, dadurch gekennzeichnet, daß die Lyophilisation der α-und/oder β-Glucosidase bei einer Temperatur durchgeführt wird, die gegen Ende der Lyophilisation 30 bis 50°C beträgt.

**Revendications**

1. Réactif sec reconstituable aux fins de diagnostic, comportant au moins un composant à base de protéine ainsi que d'autres composants, caractérisé en ce que le composant à base de protéine se présente sous forme de lyophilisat, et les autres composants sous forme de granulat.

2. Réactif selon la revendication 1 pour la détermination des lipases en utilisant 0,001 à 0,2 pourcent en poids de co-lipase et 0,2 à 10 pourcent en poids d'un triglycéride émulsifiable, caractérisé en ce que la co-lipase est présente dans le lyophilisat et le triglycéride dans le granulat.

3. Réactif selon la revendication 2, caractérisé en ce que le granulat contient en outre 10 à 60 pourcent en poids d'une substance tampon pour des pH de 6,0 à 10,5, 5 à 60 pourcent en poids de sel alcalin d'acide biliaire, 0,1 à 2,0 pourcent en poids d'agent de conservation et 0,5 à 5 pourcent en poids d'activateur.

4. Réactif selon la revendication 3, caractérisé en ce qu'il y a dans le granulat 10 à 60 pourcent en poids de sel alcalin d'acide biliaire, et que le sel alcalin d'acide biliaire dans le granulat est formé par un mélange constitué de 1 à 40 pourcent en poids d'acide taurodésoxycholique, 2 à 20 pourcent en poids d'acide cholique et 10 à 70 pourcent en poids d'acide désoxycholique sous forme de leurs sels alcalins.

5. Réactif selon l'une des revendications 2 à 4, caractérisé en ce que l'activateur est constitué par un chlorure, de préférence un chlorure de calcium.

6. Réactif selon la revendication 1 pour la détermination de l'amylase en utilisant l'α-glucosidase et les α- et β-glucosidase et les oligosaccharides, caractérisé en ce que l'α- et/ou la β-glucosidase est présente dans le lyophilisat et les oligosaccharides dans le granulat.

7. Réactif selon la revendication 6, caractérisé en ce qu'il contient 50 à 500 U/l d'α- et/ou de β-glucosidase ainsi que 0,03 à 7 mmol/l d'oligosaccharides, respectivement rapportés au réactif reconstitué.

8. Réactif selon la revendication 6 ou 7, caractérisé en ce que le granulat contient en plus 3 à 50 pourcent en poids de substance tampon pour un pH de 6 à 10, et 0,5 à 5 pourcent en poids d'activateur.

9. Réactif selon l'une des revendications 6 à 8 pour déterminer l'amylase au moyen de test UV, caractérisé en ce qu'il y a dans le lyophilisat, en outre, de l'hexokinase et de la glucose-6-phosphate-déshydrogénase, et que dans le granulat, il y a en outre du 5'-triphosphate d'adénosine.

10. Réactif selon l'une des revendications 6 à 8 pour déterminer l'amylase au moyen du test des couleurs, caractérisé en ce que l'oligosaccharide contient un groupe chromophore.

11. Réactif selon la revendication 10, caractérisé en ce que l'oligosaccharide est constitué de 4 à 10 unités de glucose et comporte du p-nitrophénol en tant que groupe chromophore.

12. Procédé pour fabriquer un réactif sec reconstituable selon l'une des revendications précédentes, caractérisé en ce qu'on introduit dans un réacteur le composant à base de protéine, ou la co-lipase, ou l'α-glucosidase et/ou la β-glucosidase sous forme de lyophilisat, et les autres composants ne contenant pas de protéine, sous forme de granulat, le granulat étant obtenu par dissolution, suspension ou émulsion des autres composants, suivis d'un séchage selon un autre procédé de séchage que la lyophilisation.

13. Procédé selon la revendication 12, caractérisé en ce que le lyophilisat est fabriqué par lyophilisation d'une solution, d'une suspension ou d'une émulsion du composant contenant la protéine, ou de la co-lipase, ou de l'α-glucosidase et/ou de la β-glucosidase dans le réacteur et le granulat est pesé et ajouté.

14. Procédé selon la revendication 12 ou 13, caractérisé en ce que le séchage des autres composants s'effectue sous vide à température ambiante ou à température plus élevée.

15. Procédé selon la revendication 12 pour fabriquer un réactif pour déterminer l'amylase, caractérisé en ce que la lyophilisation de l'α-glucosidase et/ou de la β-glucosidase est effectuée à une température qui est comprise entre 30 et 50°C vers la fin de la lyophilisation.

## Claims

1. A reconstitutable dry reagent for diagnostic purposes with at least one protein containing component and other components, characterized in that the protein containing component is present as lyophilizate and the other components as granulates.

2. The reagent according to claim 1 for the determination of lipase by using between 0,001 and 0,2 percent by weight of an emulsive triglyceride, characterized in that the co-lipase is present in the lyophilizate and the triglyceride in the granulates.

3. The reagent according to claim 2, characterized in that the granulates contain in addition 10 to 60 percent by weight of a buffer substance for pH 6,0 to 10,5, 5 to 60 percent by weight of an alkali metal bile acid salt, 0,1 to 2,0 percent by weight of a preserving agent and 0,5 to 5 percent by weight of an activator.

4. The reagent according to claim 3, characterized in that 10 to 60 percent by weight alkaline metal bile acid salt are present in the granulates and the alkaline metal bile acid salt in the granulates is formed by a mixture of 1 to 40 percent by weight taurodesoxycholic acid, 2 to 20 percent by weight cholic acid and 10 to 70 percent by weight desoxycholic acid in the form of their alkali metal salts.

5. The reagent according to one of the claims 2 to 4, characterized in that the activator is formed by a chloride, preferably calcium chloride.

6. The reagent according to claim 1 for the determination of amylase by using α-glucosidase or α- and β-glucosidase and oligosaccharides, characterized in that the α- and/or β-glucosidase is present in the lyophilizate and the oligosaccharide in the granulates.

7. The reagent according to claim 6, characterized in that it contains 50 to 500 U/l α- and/or β-glucosidase and 0,03 to 7 mmol/l oligosaccharides, based on the reconstitutable reagent in each case.

8. The reagent according to claim 6 or 7, characterized in that the granulates contain in addition 3 to 50 percent by weight of a buffer substance for pH 6 to 10 and 0,5 to 5 percent of an activator.

9. The reagent according to one of the claims 6 to 8 for the determination of amylase via UV-test, characterized in that in addition hexokinase and glucose-6-phosphate dehydrogenase are present in the lyophilizate and the granulates contain in addition adenosin-5'-triphosphate.

10. The reagent according to one of the claims 6 to 8 for determination of amylase via colour test, characterized in that the oligosaccharide has a chromophore group.

11. The reagent according to claim 10, characterized in that the oligosaccharide consists of 4 to 10 glucose units and has p-nitrophenol as chromophore group.

12. A process for making a reconstitutable dry reagent according to one of the preceding claims, characterized in that a reaction vessel is charged with the protein containing component, the colipase or the α- and/or β-glucosidase as lyophilizate and with the other components as

9         **0 134 291**         10

granulates which contain no proteins, obtaining the granulates by dissolving, suspending or emulsifying the other components and drying same by another drying process as lyophilization.

13. The process according to claim 12, characterized in that the lyophilizate is made by lyophilization of a solution, suspension or emulsion of the protein containing component, the co-lipase or the α- and/or β-glucosidase, respectively, in a reaction vessel and the granulates are added by weighing.

14. The process according to claim 12 or 13, characterized in that the drying of the other components is performed in vacuo at room temperature or elevated temperature.

15. The process according to claim 12 for making a reagent for the determination of amylase, characterized in that the lyophilization of the α- and/or β-glucosidase is performed at a temperature which is 30 to 50°C during the end period of the lyophilization.